# EUROPEAN PATENT APPLICATION

(11) **EP 3 005 947 A1**
(43) Date of publication of application: **13.04.2016**
(21) Application number: 14804133.8
(22) Date of filing: 23.05.2014
(51) Int. Cl.: A61B 8/06, A61B 8/08

(54) **ULTRASOUND IMAGE PICKUP APPARATUS AND ULTRASOUND IMAGE PICKUP METHOD**

(30) Priority: 27.05.2013 JP 2013110913
(71) Applicant: Hitachi Aloka Medical, Ltd., Mitaka-shi, Tokyo 181-8622 (JP)
(72) Inventor: CHONO, Tomoaki, Mitaka-shi Tokyo 181-8622 (JP); MORI, Osamu, Mitaka-shi Tokyo 181-8622 (JP)
(74) Representative: Strehl Schübel-Hopf & Partner
(86) International application number: PCT/JP2014/063756
(87) International publication number: WO 2014/192663

(57) **Abstract**

The invention provides an ultrasound image pickup apparatus and an ultrasound image pickup method which can evaluate the stability of Doppler information acquired by Doppler ultrasonography and can observe a moving state of biological tissues (heart or a blood vessel) of an object based on the stabilized Doppler information. The ultrasound image pickup apparatus of the invention includes: a probe which transmits and receives an ultrasound signal to and from an object; a Doppler information generation unit which generates Doppler information from the ultrasound signal; a degree of approximation calculation unit which calculates a degree of approximation of the plurality of Doppler information items; and an approximate Doppler information acquisition unit which acquires the Doppler information having a predetermined degree of approximation as approximate Doppler information.

## Description

### Technical Field

The present invention relates to an ultrasound image pickup apparatus and an ultrasound image pickup method and particularly to an ultrasound image pickup apparatus and an ultrasound image pickup method which acquire Doppler information.

### Background Art

In ultrasonography, the ultrasonography may be performed by applying stress to an object with medicine or a motion to acquire ultrasound images before and after applying stress, and observing a moving state of biological tissues (the heart or the blood vessel) of the object. In stress echocardiography, a moving state of a part of the heart is evaluated before and after applying stress, and heart diseases such as myocardial infarction are determined.

In stress echocardiography, anultrasound Doppler diagnosis device on which a coronary reserve capacity evaluation support system in which blood flow information acquired using Doppler ultrasonographycan be used as an index for indicating cardiac function, the burden of an operator when evaluating coronary reserve capacity in transthoracic echocardiography can be reduced, and the throughput can be increased by reducing the coronary reserve capacity diagnosis time, has been proposed (for example, see PTL 1).

### Citation List

### Patent Literature

[PTL 1]Japanese Patent No. 2863624

### Summary of Invention

### Technical Problem

The ultrasound Doppler diagnosis device disclosed in PTL 1 adjusts a plurality of waveforms of blood flow velocities before and after drug administration using a predetermined velocity range as a reference, displays the adjusted plurality of waveforms of blood flow velocities using the predetermined velocity range, but cannot evaluate the stability of Dopplerinformation such as the waveforms of blood flow velocities.

The invention provides an ultrasound image pickup apparatus and an ultrasound image pickup method which can evaluate the stability of Dopplerinformation acquired by Doppler ultrasonography and can observe a moving state of biological tissues (heart or a blood vessel) of an object based on the stabilized Dopplerinformation.

### Solution to Problem

An ultrasound image pickup apparatus of the invention includes: a probe which transmits and receives an ultrasound signal to and from an object; a Dopplerinformation generation unit which generates Dopplerinformation from the ultrasound signal; a degree of approximation calculation unit which calculates a degree of approximation of the plurality of Dopplerinformation items; and an approximate Dopplerinformation acquisition unit which acquires theDopplerinformation having a predetermined degree of approximation as approximate Dopplerinformation.

### Advantageous Effects of Invention

According to the invention, it is possible to evaluate the stability of Dopplerinformation acquired by the Doppler ultrasonography and to observe a moving state of biological tissues (heart or a blood vessel) of an object based on the stabilized Dopplerinformation.

### Brief Description of Drawings

Fig. 1 is a block diagram showing an ultrasound image pickup apparatus according to an embodiment.
Fig. 2 is a flowchart showing movement of the ultrasound image pickup apparatus.
Fig. 3 is a diagram showing an example of a display screen of a display unit of the ultrasound image pickup apparatus of the embodiment.
Fig. 4 is a diagram showing an example of a display screen of a display unit of the ultrasound image pickup apparatus of the embodiment.
Fig. 5 is a block diagram showing a modification example of a deformation warning unit of the ultrasound image pickup apparatus of the embodiment.
Fig. 6 is a diagram showing an example of a display screen on which approximate Dopplerinformation of each stage is displayed.
Fig. 7 is a diagram showing a change of a location of a heartbeat when acquiringDopplerinformation.
Fig. 8 is a diagram showing two types of Doppleroperations performed by a Doppler information generation unit and generation of Dopplerinformation.
Fig. 9 is a diagram showing an example of a display screen on which approximate Dopplerinformation of each stage is displayed, when Dopplerinformation is generated from the plurality of locations of ultrasound signals.

### Description of Embodiments

Hereinafter, an ultrasound image pickup apparatus according to an embodiment of the invention will be described with reference to the drawings. The ultrasound image pickup apparatus according to the embodiment of the invention includes: a probe which transmits and receives an ultrasound signal to and from an object, a Dopplerinformation generation unit which generates Dopplerinformation from the ultrasound signal, a degree of approximation calculation unit which calculates a degree of approximation of the plurality of Dopplerinformation items, and an approximate Dopplerinformation acquisition unit which acquires the plurality of Dopplerinformation items having a predetermined degree of approximation as approximate Dopplerinformation.

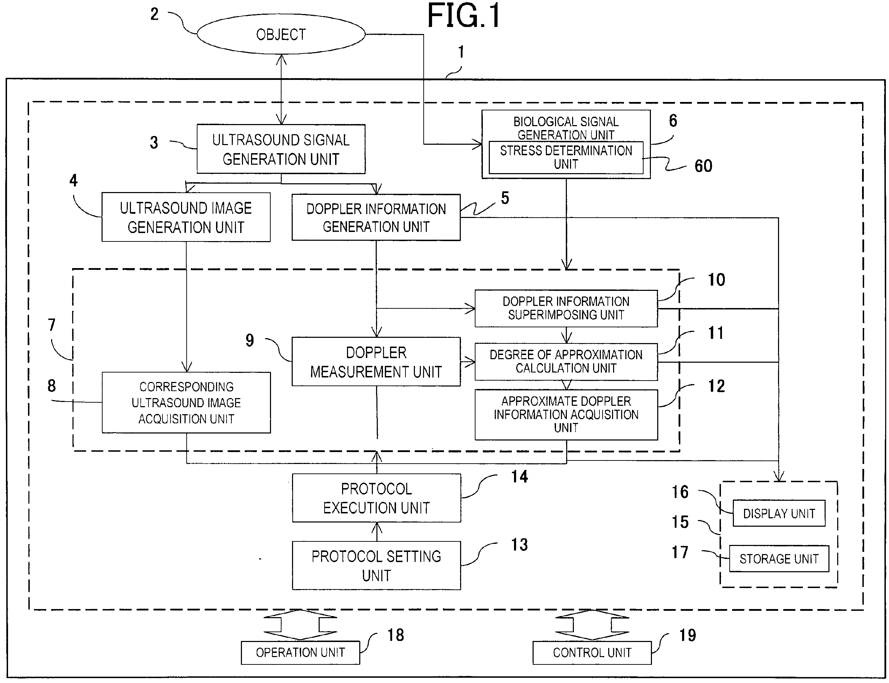

Fig. 1 is a block diagram showing the ultrasound image pickup apparatus according to the embodiment. As shown in Fig. 1, an ultrasound image pickup apparatus 1 includes an ultrasound signal generation unit 3, an ultrasound image generation unit 4, a Dopplerinformation generation unit 5, a biological signal generation unit 6, a data acquisition unit 7, a protocol setting unit 13, a protocol execution unit 14, an output unit 15, an operation unit 18, and a control unit 19.

The biological signal generation unit 6 includes a stress determination unit 60. The data acquisition unit 7 includes a corresponding ultrasound image acquisition unit 8, a Doppler measurement unit 9, a Dopplerinformation superimposing unit 10, a degree of approximation calculation unit 11, and an approximate Dopplerinformation acquisition unit 12. The output unit 15 includes a display unit 16 and a storage unit 17.

The ultrasound signal generation unit 3 generates an ultrasound signal of a biological tissue of an object 2 through the probe and an ultrasound signal transmission and reception unit. The probe transmits and receives an ultrasound signal from a vibrator to a target tissue of the object 2. The probe has a linear, convex, or sector scanning function. The ultrasound signal transmission and reception unit transmits and receives an electrical ultrasound signal to and from the probe. The ultrasound signal generation unit 3 receives information relating to power or timing of the transmission and reception from the control unit 19 and is controlled so as to acquire a desired ultrasound signal. The ultrasound signal generation unit 3 performs signal processing according to an imaging setting, with respect to the ultrasound signal received from the ultrasound signal transmission and reception unit by a phasing circuit or an amplifying circuit.

The ultrasound image generation unit 4 generates an ultrasound image from the ultrasound signal based on the imaging setting of the apparatus (for example, a scanning range of an ultrasound beam or a gain setting). The ultrasound image generation unit 4 generates amplitude information regarding the ultrasound signal and performs imaging. The ultrasound image or the amplitude information is updated in real time according to a frame rate, stored in a storage medium of the storage unit 17, and displayed on a screen as a moving image by the display unit 16.

The Dopplerinformation generation unit 5 performs a Doppler operation of a part set by the operation unit 18 and generates Dopplerinformation. The Doppler operation is performed in modes of pulsed Doppler, continuous wave Doppler, color Doppler, power Doppler, and tissue Doppler. The Dopplerinformation generation unit 5 generates an image of the Dopplerinformation acquired from the Doppler operation. For example, a velocity component value acquired from the Doppler operation is converted into a luminance value and generates an image thereof. The Dopplerinformation is updated in real time and stored in the storage medium of the storage unit 17, and displayed on a screen as a moving image by the display unit 16.

The biological signal generation unit 6 receives a biological signal (signal of electrocardiogram or phonocardiogram) of the object 2, converts the biological signal into biological signal data, displays the biological signal data on a screen by the display unit 16, and stores the biological signal data in the storage unit 17. The biological signal data controls operation timing of the data acquisition unit 7. For example, the data acquisition unit 7 acquires an ultrasound image or Dopplerinformation for each R wave of an electrocardiogram. In addition, the stress determination unit 60 determines stress based on the biological signal and determines each stage of a REST (before applying stress) stage, a stress stage 1 (STAGE1), a stress stage 2 (STAGE2), ..., and a POST (after applying stress) stage. The data acquisition unit 7 acquires an ultrasound image or Dopplerinformation for each stage.

The data acquisition unit 7 acquires an image or a measurement value necessary for stress echocardiography. The data acquisition unit 7 follows a protocol (procedure) executed by the protocol execution unit 14. In stress echocardiography, a protocol (procedure) for acquiring a plurality of ultrasound images (sectional images) is set in each stage of the REST (before applying stress) stage, the stress stage 1, the stress stage 2, ..., and the POST (after applying stress) stage, and the data acquisition unit 7 acquires an image or data according to the protocol (procedure). The ultrasound images (sectional images) of the embodiment includes four types of an apical 2 chamber A2C (apical two chamber view), apical 4 chamber A4C (apical four chamber view), a parasternal short axis view SAX (short axis view), and a parasternal long axis view LAX (long axis view). A moving state of a part of the heart is evaluated based on each ultrasound image (each sectional image) and heart diseases such as myocardial infarction are determined. For example, after acquiring each ultrasound image (each sectional image), a moving state of a part of the myocardium is scored while observing a moving image, an index wall motion score index(WMSI) indicating a state of the movement of the entire myocardium using the scores, and heart diseases such as myocardial infarction are determined. In stress echocardiography, blood flow information acquired using Doppler ultrasonography is used as an index indicating a cardiac function, a mitral orifice blood flow velocity waveform acquired by a pulse Doppler system and a mitral annulus exercise velocity waveform acquired by a tissue Doppler system of the left ventricle are used for diagnosis of heart failure (systolic dysfunction or diastolic dysfunction).

The data acquisition unit 7 is controlled by the control unit 19 so as to acquire an image or a measurement value according to the timing of the biological signal data generated by the biological signal generation unit 6. The acquired image or measurement value is transmitted to the output unit 15, displayed by the display unit 16, and stored by the storage unit 17.

The corresponding ultrasound image acquisition unit 8 follows a protocol (procedure) executed by the protocol execution unit 14. The corresponding ultrasound image acquisition unit 8 acquires a corresponding ultrasound image corresponding to the approximate Dopplerinformation according to the timing of the biological signal data generated by the biological signal generation unit 6. The corresponding ultrasound image acquisition unit 8, for example, acquires the corresponding ultrasound image for one heart beat (R-R wave) from an R wave to the next R wave of an electrocardiogram which is the biological signal data. The corresponding ultrasound image includes an image showing an amplitude value such as a B mode image or an M mode image and includes an image obtained by superimposing Dopplerinformation of color Doppler on the ultrasound image. The corresponding ultrasound image acquisition unit 8 is synchronized at the timing when the approximate Dopplerinformation acquisition unit 12 acquires the approximate Dopplerinformation and acquires an ultrasound imagecorresponding to the approximate Dopplerinformation as the correspondingultrasound image. That is, the correspondingultrasound image and the approximate Dopplerinformation are acquired at the synchronized timing. The acquired correspondingultrasound image is transmitted to the output unit 15, displayed by the display unit 16, and stored by the storage unit 17.

The Doppler measurement unit 9 calculates the measurement value representing a moving state of a biological tissue (heart or blood vessel) of the object 2 and showing useful characteristics for diagnosis, based on the Dopplerinformation. For example, when calculating the measurement value based on the mitral orifice blood flow velocity waveform in the heart (Dopplerinformation), measurement values of an E (early diastole) wave velocity, an A (atrial systole) wave velocity, and a DT (E wave damping time) are calculated. In this case, it is necessary to set an apex of the E wave, an apex of the A wave, and a damping width of the E wave, in order to calculate these measurement values. By using an automatic tracing function of the Doppler waveform, the location where the Dopplerinformation is acquired may be automatically set and the measurement values may be calculated based on theDopplerinformation. The measurement values may be calculated based on theDopplerinformation by using the Doppler automatic tracing function in real time, while performing the scanning in the Doppler mode. The calculated measurement values are transmitted to the degree of approximation calculation unit 11 and the output unit 15 and are used for determination of stability of the degree of approximation calculation unit 11.

TheDopplerinformation superimposing unit 10 superimposes the plurality of Dopplerinformation items and displays the information on the display unit 16. The Dopplerinformation superimposing unit 10 superimposes the plurality of Dopplerinformation items corresponding to the plurality of heartbeats. When superimposing the Dopplerinformation items for three heartbeats using the R-R wave as a reference, the Dopplerinformation superimposing unit 10 superimposes the plurality of Dopplerinformation items corresponding to a first, a second, and a third heartbeat. In this case, the Dopplerinformation superimposing unit 10 makes each start time of the R wave coincide with each other and superimposes the plurality of Dopplerinformation items. In addition, the Dopplerinformation superimposing unit 10 may make the start time of the R wave and the finish time of the R wave coincide with each other and may superimpose the plurality of Dopplerinformation items. For example, the Dopplerinformation superimposing unit 10 may enlarge or contract the Doppler waveform of the Dopplerinformation in a time axis direction and superimpose the plurality of Dopplerinformation items, in order to make the start time of the R wave and the finish time of the R wave coincide with each other. As a method of superimposing the plurality of Dopplerinformation items and displaying the information items on the display unit 16, the Dopplerinformation superimposing unit 10 may superimpose the plurality of Dopplerinformation items and display the information items on the display unit 16, by displaying an average value of the plurality of Dopplerinformation items, displaying by respectively changing colors of the plurality of Dopplerinformation items, or displaying by respectively changing the transparency of the plurality of Dopplerinformation items. In addition, the Dopplerinformation superimposing unit 10 may superimpose the Dopplerinformation items acquired in real time and display the information items on the display unit 16, to update the plurality of superimposed Dopplerinformation items. In this case, when new Dopplerinformation is input, the oldest (firstly input) superimposed Dopplerinformation is removed and the plurality of superimposed Dopplerinformation items are updated. Accordingly, the plurality of Dopplerinformation items are updated in real time. The plurality of superimposed Dopplerinformation items are transmitted to the output unit 15, displayed by the display unit 16, and stored by the storage unit 17.

The degree of approximation calculation unit 11 calculates a degree of approximation of the plurality of Dopplerinformation items. The degree of approximation calculation unit 11 determines whether or not the plurality of Dopplerinformation items are approximate without being disordered, for each heartbeat, based on the degree of approximation. For example, when the heartbeat is not stable such as an irregular pulse, the Dopplerinformation changes for each heartbeat, and accordingly, reliability of a moving state of biological tissues (heart or a blood vessel) of the object based on the Dopplerinformation decreases. Thus, in order to properly grasp the moving state of the biological tissues, it is necessary to acquire the stable Dopplerinformation.

The degree of approximation calculation unit 11 calculates at least one degree of approximation of a Doppler waveform of the plurality of Dopplerinformation items synchronized with the biological signal, the time of the plurality of Dopplerinformation items, and the measurement value of the plurality of Dopplerinformation items. The degree of approximation calculation unit 11 sets a reference for calculating the degree of approximation, calculates the degree of approximation of the Dopplerinformation based on at least one of a difference, a ratio, a correlation coefficient, and pattern matching to the reference, and determines the stability of the Dopplerinformation based on the degree of approximation of the Dopplerinformation. For example, when calculating the degree of approximation based on the blood flow information of the object 2, the degree of approximation calculation unit 11 enlarges or contracts the waveforms of blood flow velocities (Dopplerinformation) for making the start time and the finish time of the R-R wave coincide with each other, and determines the stability of the Dopplerinformation with the fact whether or not at least one of a difference, a ratio, a correlation coefficient, and pattern matching results of the plurality of waveforms of blood flow velocities (Dopplerinformation) is within a threshold value (or equal to or greater than a predetermined threshold value). In this case, the Dopplerinformation superimposing unit 10 superimposes the plurality of waveforms of blood flow velocities (Dopplerinformation) based on the R-R wave and displays the waveforms of blood flow velocities on the display unit 16.

In addition, when calculating the degree of approximation of the time of the plurality of Dopplerinformation items, the degree of approximation calculation unit 11 may determine the stability of the difference, a ratio, a correlation coefficient, and pattern matching results of the measurement value calculated by the Doppler measurement unit 9 based on the plurality of Doppler information items is within a threshold value (or equal to or greater than a predetermined threshold value). In this case, the Doppler information superimposing unit 10 displays the measurement value calculated by the Doppler measurement unit 9 based on the plurality of Doppler information items on the display unit 16.

In addition, the degree of approximation calculation unit 11 may determine the stability of the Doppler information based on an index (index indicating stability of the Doppler information) input by an examiner using the Doppler information displayed on the display unit 16 by the Doppler information superimposing unit 10.

The degree of approximation calculated by the degree of approximation calculation unit 11 is transmitted to the output unit 15, displayed by the display unit 16, and stored by the storage unit 17.

The approximate Doppler information acquisition unit 12 acquires the plurality of Doppler information items having a predetermined degree of approximation as approximate Doppler information having the determined stability, based on the degree of approximation calculated by the degree of approximation calculation unit 11, and stores the Doppler information items in the storage unit 17. The approximate Doppler information acquisition unit 12 performs synchronization with the corresponding ultrasound image acquired by the corresponding ultrasound image acquisition unit 8 and acquires the Doppler information corresponding to the ultrasound image. That is, the corresponding ultrasound image and the Doppler information at the same time are stored in the storage unit 17.

The storage unit 17 stores data items such as the ultrasound image, the amplitude information, the biological signal data, the Doppler information, the measurement value based on the Doppler information, and the degree of approximation in a storage medium, while updating the data items in real time. In this case, when new data is input, the oldest (firstly input) data may be removed and the updated data may be stored. For example, when the number of data items of 1 set stored by the storage unit 17 is set by the protocol setting unit 13 in advance, the storage unit 17 stores data items for 3 heartbeats in 1 set, and data regarding a fourth heartbeat is input, and thus the oldest 1 heartbeat is removed. That is, the storage unit 17 may operate so as to sequentially remove old data and sequentially add new data using a first-in first-out system.

In addition, the storage unit 17 may store the plurality of Doppler information items which are most approximate to the reference (reference for calculating the degree of approximation) set by the degree of approximation calculation unit 11, as the approximate Doppler information. Further, the storage unit 17 may store the plurality of Doppler information items of which a difference from the reference (reference for calculating the degree of approximation) set by the degree of approximation calculation unit 11 is within a predetermined threshold value, as the approximate Doppler information.

The protocol setting unit 13 sets the acquisition order of the images or data items in the stress echocardiography. For example, in the test using the B mode image (sectional image), the protocol setting unit 13 programs each stage of the REST (before applying stress) stage, the stress stage 1, the stress stage 2, ..., and the POST (after applying stress) stage so that A2C, A4C, SAX, and LAX are acquired. For example, the protocol setting unit 13 programs each stage so that, first, the data items are acquired in the order of A2C, A4C, SAX, and LAX in the REST stage, next, the data items are acquired in the same order in the stress stage 1, finally, the data items are acquired in the same order in the POST stage, and the operation ends. Since the acquisition order of the images or data items is programmed, when the image or data is acquired, the tags of each stage and the B mode image (sectional image) are automatically attached to the data. In addition, the tag of the B mode image (sectional image) may be attached to the data later, without limiting the order from A2C to LAX. Further, the acquisition order of the Doppler information items may be set. For example, when focusing on the valve, the data acquisition order regarding Tricuspid Valve TV, Mitral Valve MV, Aortic Valve AV, and Pulmonic Valve PV are set. The protocol setting unit 13 may include the B mode image and the Doppler information in the same protocol or may set a protocol for singly acquiring the Doppler information.

The protocol execution unit 14 executes the protocol set by the protocol setting unit 13. For example, when the stress echocardiography is started and the acquisition completion of A2C in the initial REST stage is confirmed, the protocol execution unit 14 displays a message on a screen so as to acquire A4C next. The protocol execution unit 14 repeatedly executes the message display in the order of protocols. In addition, after starting the applying of stress (stress stage 1 start), the protocol execution unit 14 may display a message promoting the acquisition of the image or the data on a screen, so as to acquire the data within the acquisition limitation time for each stage using a timer function.

The output unit 15 includes the display unit 16 and the storage unit 17, and the ultrasound image, the amplitude information, the biological signal data, the Doppler information, the measurement value based on the Doppler information, the degree of approximation, and the message by the protocol are stored by the storage unit 17 and output to the display unit 16.

The display unit 16 performs a process so that the plurality of data items are disposed or superimposed so as to be displayed in an easy to see manner. The display unit 16 is a display device such as a CRT or an LCD and transmits and receives data in a wired or wireless manner.

The storage unit 17 stores various data items. In addition, the storage unit 17 accommodates programs for operating various systems configuring the ultrasound image pickup apparatus 1 or measurement operation methods. The storage unit 17 includes a storage medium such as a semiconductor memory, an optical disc, or a magnetic disc. The storage medium may be an external storage medium connected through a network.

The operation unit 18 is an interface for performing various operations of the apparatus. The operation unit 18 inputs completion of the acquisition of the B mode image (sectional image) or the Doppler information in each stage, in order to promote the operation in the order of the protocols. In addition, the operation unit 18 is an interface for inputting the protocols set by the protocol setting unit 13. The operation unit 18 includes input devices such as a keyboard, a trackball, a switch, and a dial. The operation unit 18 may include a voice input device.

The control unit 19 controls the entire system and controls the timing of the acquisition of the image or the data based on the protocol set by the protocol setting unit 13. The control unit 19 performs the control operation in order to promote the operation in the order of the protocols. The control unit 19 performs the control operation of acquiring the image or the measurement value according to the timing of the biological signal data or superimposing the Doppler information items. The control unit 19 includes a control device such as a CPU.

Next, the operation of the ultrasound image pickup apparatus 1 and an ultrasound image pickup method according to the embodiment will be described using a flowchart. The ultrasound image pickup method includes: transmitting and receiving an ultrasound signal to and from an object; generating Doppler information from the ultrasound signal; calculating a degree of approximation of the plurality of Doppler information items; and acquiring the Doppler information having a predetermined degree of approximation as approximate Doppler information.

In the embodiment, the ultrasound image pickup apparatus 1 (Doppler measurement unit 9) calculates the measurement value indicating the moving state of the biological tissues (heart or a blood vessel) of the object based on the Doppler information for each stage. In addition, the ultrasound image pickup apparatus 1 (degree of approximation calculation unit 11) determines the stability of the Doppler information based on the degree of approximation of the Doppler information in the stress echocardiography. Further, the ultrasound image pickup apparatus 1 displays the plurality of Doppler information items on the display unit 16 as the Doppler image in real time.

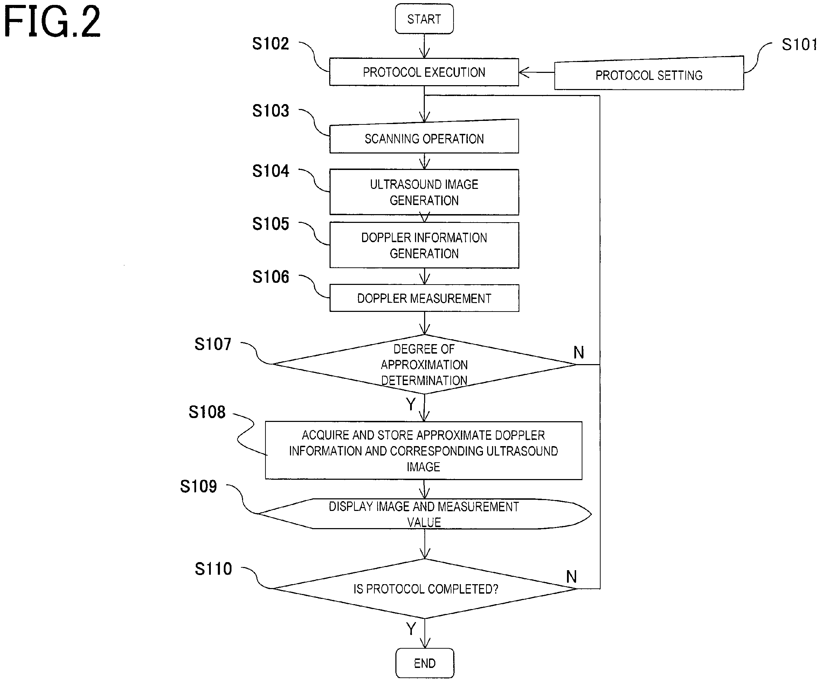

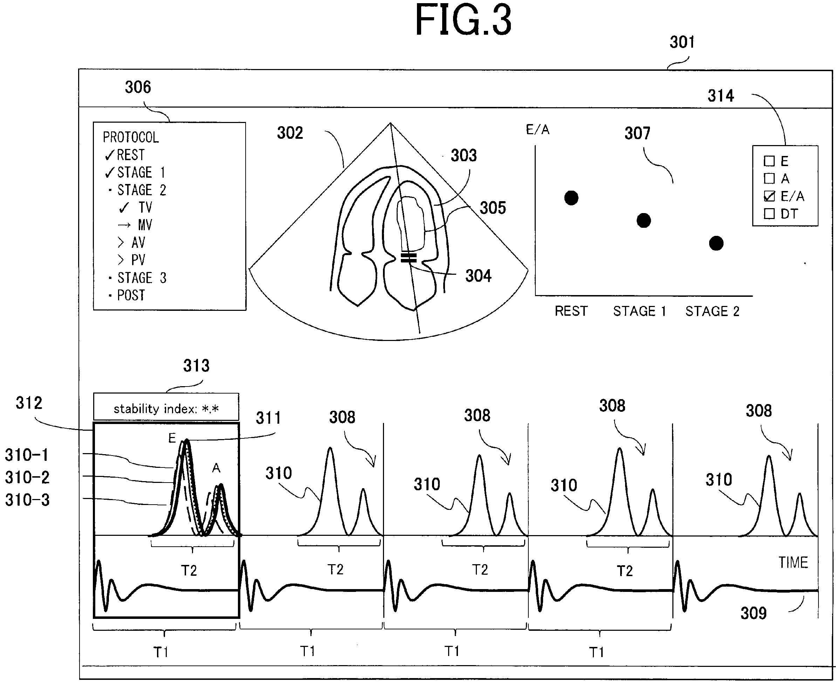

Fig. 2 is a flowchart showing the movement of the ultrasound image pickup apparatus 1. In Step S101, the protocol setting unit 13 sets the protocols in advance by using the operation unit 18. The acquisition order of the images or data items is set in the protocol. For example, the types of images acquired in each stage of the REST (before applying stress) stage, the stress stage 1, the stress stage 2, ..., and the POST (after applying stress) stage are set. Fig. 3 is a diagram showing an example of a display screen of the display unit 16 of the ultrasound image pickup apparatus 1 of the embodiment. As shown in a protocol list 306 on the display screen of Fig. 3, the protocol is set so that each Doppler image of TV, MV, AV, and PV is acquired in each stage of the REST (before applying stress) stage, the stress stage 1, the stress stage 2, the stress stage 3, and the POST (after applying stress) stage.

In Step S102, the protocol execution unit 14 executes the protocol according to the protocol set by the protocol setting unit 13. When the acquisition of the image or the data is completed, the protocol execution unit 14 may display a message promoting the acquisition of the next image or data (protocol list or the like) on a screen and promotes the operation in the acquisition flow of the image or data. The protocol list 306 of Fig. 3 presents the progressing state of the protocol to the examiner by displaying check marks in a case where acquisition of the image or the data is completed and displaying arrow marks in a case where the acquisition of the image or the data becomes progressed. For example, the protocol list 306 of Fig. 3 presents that the acquisition of each Doppler image of TV, MV, AV, and PV is completed in the REST (before applying stress) stage and the stress stage 1, and the acquisition of the Doppler image of TV is completed and the acquisition of the Doppler image of MV becomes progressed in the stress stage 2.

In Step S103, the Doppler image is scanned in order to acquire the Doppler information presented by the protocol list 306. Since the protocol list 306 of Fig. 3 presents that the acquisition of the Doppler image of MV becomes progressed in the stress stage 2, the examiner scans the Doppler image of MV by placing a Doppler cursor 304 to the position of MV, while operating the operation unit 18 and scanning a B mode image (sectional image) 302 of a heart 303 by bringing the probe into contact with a body surface.

In Step S104, the ultrasound image generation unit 4 generates an ultrasound image (B mode image or sectional image) 302 of the heart 303. The generated ultrasound image (B mode image or sectional image) 302 is displayed on a screen 301 of the ultrasound image pickup apparatus 1 by the display unit 16. In this case, the ultrasound image generation unit 4 generates the ultrasound image (B mode image or sectional image) 302 from the ultrasound signal and performs imaging, based on the scanning setting of the apparatus (for example, a scanning range of an ultrasound beam or a gain setting). The ultrasound image (B mode image or sectional image) 302 or the amplitude information is updated in real time according to a frame rate, stored in a storage medium of the storage unit 17, and displayed on the screen 301 as a moving image by the display unit 16.

When scanning the Doppler image in the color Doppler operation, the Doppler information generation unit 5 may superimpose a color Doppler image 305 which is Doppler information on the ultrasound image (B mode image or sectional image) 302 and displays the image on the screen 301 as a moving image by the display unit 16.

In Step S105, the Doppler information generation unit 5 performs the Doppler operation of a part set by the operation unit 18 and generates the Doppler information (Doppler image) to perform the imaging. The Doppler information generation unit 5 performs the Doppler operation of a part set by the Doppler cursor 304 by the operation unit 18. In the embodiment, a case of operating velocity distribution (blood flow information or tissue exercise information) 308 which is Doppler information of the location of the Doppler cursor 304 performed by the Doppler information generation unit 5 by a pulse Doppler system will be described. In the Doppler image (Doppler information) 300 of the velocity distribution, a horizontal axis indicates time, a vertical axis indicates velocity, and imaging is performed by allocating high luminance in a location of velocity distribution with a large number of velocity components and allocating low luminance in a location of velocity distribution with a small number of velocity components. In the Doppler image (Doppler information) 300 of Fig. 3, the velocity distribution (blood flow information or tissue exercise information) 308 is displayed so as to sweep from the right to the left of the image 301 over time, and displayed so as to have the same time phase with the ultrasound image (sectional image) 302 or an electrocardiogram 309 which is the biological signal data on the same screen.

In Step S106, the Doppler measurement unit 9 calculates the measurement value representing a moving state of a biological tissue (heart or blood vessel) of the object and showing useful characteristics for diagnosis, based on the Doppler information. Herein, the measurement value of the Doppler measurement by Doppler automatic tracing will be described. In the Doppler automatic tracing, a Doppler waveform (blood flow information or tissue exercise information) is acquired by performing automatic tracing of a boundary detected based on the luminance of the Doppler image (Doppler information), and in the Doppler measurement, the measurement value such as the damping time of the velocity or the maximum velocity is calculated based on a Doppler waveform (blood flow information or tissue exercise information) 310. For example, the maximum velocity is calculated by detecting a peak of the Doppler waveform (blood flow information or tissue exercise information) 310 and the measurement value such as the damping time is calculated based on a change in velocity from the peak of the Doppler waveform (blood flow information or tissue exercise information) 310. By using the automatic tracing function of the Doppler waveform, the location for acquiring the Doppler information is automatically set, an apex E of the E wave, an apex A of the A wave, and a damping width of the E wave are calculated based on the Doppler information, and the measurement values such as the E (early diastole) wave velocity, the A (atrial systole) wave velocity, and the DT (E wave damping time) are calculated. In addition, the operation unit 18 may set a cursor in the Doppler waveform (blood flow information or tissue exercise information) 310 and the Doppler measurement unit 9 may calculate the measurement value of the cursor based on the Doppler information.

The Doppler information superimposing unit 10 superimposes the plurality of Doppler information items and displays the information on the display unit 16. The Doppler information superimposing unit 10 superimposes the plurality of Doppler information items corresponding to the plurality of heartbeats. As shown in Fig. 3, a superimposing region 312 for displaying the plurality of superimposed Doppler information items is set. The Doppler information superimposing unit 10 superimposes and displays the swept Doppler information (blood flow information or tissue exercise information) items 310-1, 310-2, and 310-3 in the superimposing region 312. When superimposing the Doppler information items for three heartbeats using the R-R wave as a reference, the Doppler information superimposing unit 10 superimposes the plurality of Doppler information (blood flow information or tissue exercise information) items 310-1, 310-2, and 310-3 corresponding to a first, a second, and a third heartbeat. The sweeping direction of the velocity distribution (blood flow information or tissue exercise information) 308 is from the right to the left of the screen 301. When new Doppler information is input, the Doppler information superimposing unit 10 removes the oldest superimposed Doppler information, updates the plurality of Doppler information items to superimpose and display the information items in the superimposing region 312. As the timing of the updating, the plurality of Doppler information items may be updated each time the R wave is input, or the plurality of Doppler information items may be continuously updated each time the Doppler information (blood flow information or tissue exercise information) is input while sweeping the Doppler information. In addition, since the Doppler waveform 310 is acquired by the Doppler automatic tracing, the Doppler information superimposing unit 10 may superimpose the plurality of Doppler waveforms 310 as the Doppler information (blood flow information or tissue exercise information) items 310-1, 310-2, and 310-3. Further, the Doppler information superimposing unit 10 may calculate an average value of the plurality of superimposed Doppler information (blood flow information or tissue exercise information) items 310-1, 310-2, and 310-3 and superimpose an average Doppler information 311 on the plurality of Doppler information (blood flow information or tissue exercise information) items 310-1, 310-2, and 310-3.

The Doppler information superimposing unit 10 may make the start times of the R-R wave (biological signal) which corresponds to (is synchronized with) each of the Doppler information items 310-1, 310-2, and 310-3 coincide with each other and may superimpose a plurality of Doppler information items 310-1, 310-2, and 310-3. In addition, when the start time and the finish time of the R-R wave (biological signal) which corresponds to (is synchronized with) each of the Doppler information items 310-1, 310-2, and 310-3 are different from each other, the Doppler information superimposing unit 10 may enlarge or contract the waveform of the Doppler information items 310-1, 310-2, and 310-3 in the time axis direction, in order to make the start time of the R wave and the finish time of the R wave coincide with each other, and superimpose the plurality of Doppler information items 310-1, 310-2, and 310-3. Further, the Doppler information superimposing unit 10 may superimpose the plurality of R-R waves (biological signals) regarding the electrocardiogram 309 and displays the waves on the display unit 16, in the same manner as the Doppler information. In addition, the Doppler information superimposing unit 10 may display only the R-R wave (biological signal) for the third heartbeat lastly input, on the display unit 16.

In Step S107, the degree of approximation calculation unit 11 calculates a degree of approximation of the plurality of Doppler information items. The degree of approximation calculation unit 11 determines the stability of the Doppler information based on the degree of approximation of the Doppler information. The stability of the Doppler information is a quantified index and the approximation of the Doppler information which corresponds to (is synchronized with) the plurality of heartbeats is the condition of the stability. When the plurality of Doppler information items are approximate, it is determined that the plurality of heartbeats are approximate and in a stable state, and accordingly, it is important to acquire the stable Doppler information, in order to improve accuracy of the diagnosis.

As shown in Fig. 3, when calculating the degree of approximation based on the velocity distribution (blood flow information or tissue exercise information) 308 which is the Doppler information of the object 2, the degree of approximation calculation unit 11 determines the stability of the Doppler information with the fact whether or not a difference of the plurality of velocity distribution (blood flow information or tissue exercise information) items 308 is within a threshold value. In Fig. 3, the Doppler information superimposing unit 10 superimposes plurality of Doppler information (blood flow information or tissue exercise information) items 310-1, 310-2, and 310-3 based on the R-R wave and displays the Doppler information in the superimposing region 312. In addition, an average value of the plurality of superimposed Doppler information (blood flow information or tissue exercise information) items 310-1, 310-2, and 310-3 is calculated and the average Doppler information 311 is superimposed and displayed in the superimposing region 312. The degree of approximation calculation unit 11 calculates a difference of each of the plurality of Doppler information items 310-1, 310-2, and 310-3 as a degree of approximation and determines that the plurality of Doppler information items having a predetermined degree of approximation is the approximate Doppler information (stable Doppler information). In addition, the degree of approximation calculation unit 11 may calculate a difference between the average Doppler information 311 and the plurality of Doppler information items 310-1, 310-2, and 310-3 as a degree of approximation, and may determine that the plurality of Doppler information items having a predetermined degree of approximation is the approximate Doppler information (stable Doppler information).

In addition, when calculating a degree of approximation of the time of the plurality of Doppler information items, the degree of approximation calculation unit 11 may calculate each difference of time T1 of the R-R wave (1 heartbeat) corresponding to the Doppler information items 310-1, 310-2, and 310-3 as a degree of approximation and may determine that the plurality of Doppler information items having a predetermined degree of approximation is the approximate Doppler information (stable Doppler information). Further, the degree of approximation calculation unit 11 may calculate each difference of time T2 of the Doppler information items 310-1, 310-2, and 310-3 corresponding to the R-R wave (1 heartbeat) as a degree of approximation and may determine that the plurality of Doppler information items having a predetermined degree of approximation is the approximate Doppler information (stable Doppler information). The Doppler information superimposing unit 10 superimposes and displays the time T1 of the R-R wave (1 heartbeat) or the time T2 of the Doppler information items 310-1, 310-2, and 310-3 in the superimposing region 312.

In addition, when calculating the degree of approximation of the measurement value based on the plurality of Doppler information items, the degree of approximation calculation unit 11 may calculate a difference of the measurement value calculated by the Doppler measurement unit 9 based on the plurality of Doppler information items as a degree of approximation and determine that the plurality of Doppler information items having a predetermined degree of approximation is the approximate Doppler information (stable Doppler information). In this case, the Doppler information superimposing unit 10 displays the measurement value calculated by the Doppler measurement unit 9 based on the plurality of Doppler information items on the display unit 16. In Fig. 3, the measurement value is displayed in a measurement value region 307. The displayed measurement value is selected from a measurement value list 314. Since the measurement value is stored in each stage by the automatic measurement of the Doppler waveform by the Doppler automatic tracing, the plot in the measurement value region 307 is added each time the protocol proceeds.

As described above, the degree of approximation calculation unit 11 calculates the degree of approximation of the Doppler information based on the difference of the plurality of Doppler information items and determines the stability of the Doppler information based on the degree of approximation of the Doppler information. For example, the stability of the Doppler information may be determined with the fact whether or not the difference of the plurality of Doppler information items is within a threshold value. The difference of the plurality of Doppler information items includes a total value such as a deviation of the plurality of Doppler information items, in addition to the subtraction value of the plurality of Doppler information items.

In addition, the degree of approximation calculation unit 11 may determine the stability of the Doppler information based on the index (index indicating stability of the Doppler information) input by an examiner using the Doppler information displayed on the display unit 16 by the Doppler information superimposing unit 10. The degree of approximation and determined results of the stability are displayed in a stability index region 313 on the screen.

In Step S107, when it is determined that the Doppler information is stable, the process proceeds to Step S108. In Step S108, the approximate Doppler information acquisition unit 12 acquires the Doppler information having a predetermined degree of approximation as the approximate Doppler information having the determined stability. For example, the approximate Doppler information acquisition unit 12 acquires the Doppler information (blood flow information or tissue exercise information) items 310-1, 310-2, and 310-3 for three heartbeats of Fig. 3 as 1 set. In this case, the corresponding ultrasound image acquisition unit 8 is synchronized at the timing when the approximate Doppler information acquisition unit 12 acquires the approximate Doppler information and acquires the corresponding ultrasound image (ultrasound image 302) corresponding to the approximate Doppler information. That is, the corresponding ultrasound image and the Doppler information at the same time are stored in the storage unit 17.

In addition, since the approximate Doppler information items are approximate to each other, the approximate Doppler information acquisition unit 12 may acquire one of the plurality of approximate Doppler information items. For example, the approximate Doppler information acquisition unit 12 may acquire one of the Doppler information (blood flow information or tissue exercise information) items 310-1, 310-2, and 310-3 for three heartbeats of Fig. 3. The Doppler information (blood flow information or tissue exercise information) to be acquired may be previously set.

In Step S107, when it is not determined that the Doppler information is stable, the process returns to Step S103. In this case, the Doppler image is scanned until the stable Doppler information is acquired.

In Step S109, the approximate Doppler information (including the plurality of superimposed approximate Doppler information) and the corresponding ultrasound image are transmitted to the output unit 15, displayed by the display unit 16, and stored by the storage unit 17. When the Doppler information items 310-1, 310-2, and 310-3 of Fig. 3 are approximate to each other, the plurality of Doppler information items 310-1, 310-2, and 310-3 are superimposed as the approximate Doppler information, transmitted to the output unit 15, displayed by the display unit 16, and stored by the storage unit 17. In this case, the plurality of sets from the set having highest degree of approximation may be transmitted to the output unit 15 and displayed by the display unit 16. The display unit 16 displays the plurality of sets of the approximate Doppler information in the descending order of the degree of approximation. The approximate Doppler information (including the plurality of superimposed approximate Doppler information items) stored by the storage unit 17 and the corresponding ultrasound image are read out after the protocol completion and are used in ex-post diagnosis.

In Step S110, the protocol execution unit 14 determines the completion of the protocol. The protocol execution unit 14 completes the image acquisition of stress echocardiography, when the acquisition of the final image or data is completed according to the protocol list 306 set by the protocol setting unit 13. When the acquisition of the final image or data is not completed, the operation proceeds to the next protocol and the protocol execution unit 14 continues the acquisition of the image or the data.

According to the embodiment, it is possible to acquire the ultrasound image and the Doppler information at the same time according to the protocol, to evaluate the stability of the Doppler information, and to observe the moving state of the biological tissues (heart or a blood vessel) of the object based on the stable approximate Doppler information. In addition, it is possible to observe the moving state of the biological tissues (heart or a blood vessel) of the object based on the stable approximate Doppler information in real time. For example, it is possible to acquire the stable approximate Doppler information in a state of stable heartbeat in real time and to observe the moving state of the heart in real time. In addition, it is possible to acquire the ultrasound image and the measurement value (Doppler information) at the same time by the Doppler automatic tracing and to observe the moving state of the biological tissues (heart or a blood vessel) of the object for each stage based on the stable approximate Doppler information, by displaying a graph of the approximate measurement value (approximate Doppler information) for each stage of stress echocardiography.

Hereinabove, the embodiment according to the invention has been described, but the invention is not limited thereto and changes and modifications can be performed within a range disclosed in claims.

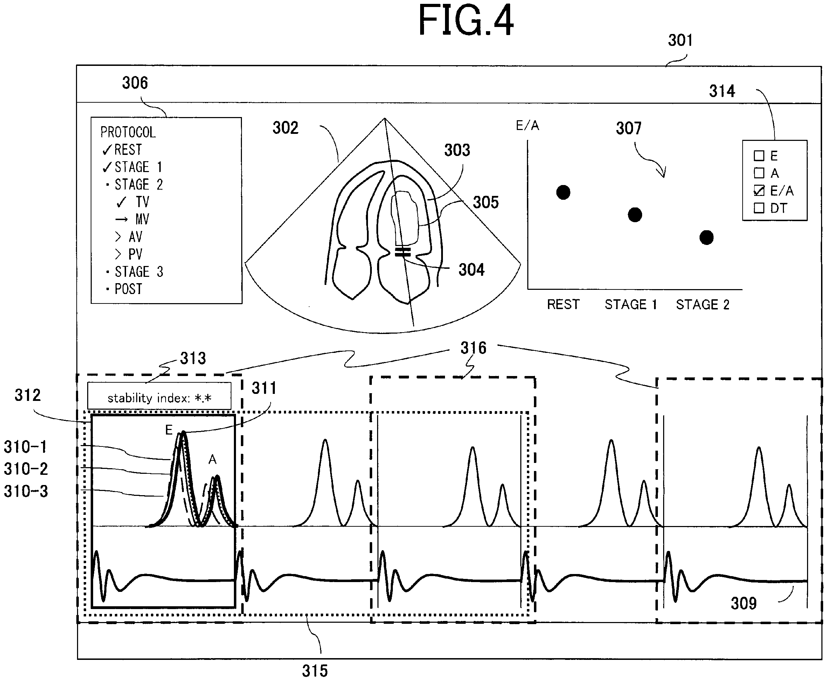

For example, in Fig. 3, the plurality of Doppler information items are continuously updated and superimposed and displayed in the superimposing region 312, but as shown in Fig. 4, the Doppler information superimposing unit 10 may superimpose the plurality of Doppler information items having a predetermined degree of approximation and display the information items on the display unit 16. In addition, the Doppler information superimposing unit 10 may superimpose the plurality of Doppler information items corresponding to intervals of predetermined heartbeats and display the information items on the display unit 16. In the embodiment, the Doppler information superimposing unit 10 may update a plurality of (for example, for three heartbeats) continuous Doppler information items 315 and superimpose and display the Doppler information items in the superimposing region 312, may update a plurality of (for example, for three heartbeats) Doppler information items 316 having a predetermined degree of approximation and superimpose and display the Doppler information items in the superimposing region 312, or may update a plurality of (for example, for three heartbeats) Doppler information items 316 corresponding to intervals of predetermined heartbeats (for example, for two heartbeats) and superimpose and display the Doppler information items in the superimposing region 312. As shown in Fig. 4, the Doppler information superimposing unit 10 sets the Doppler information (Doppler waveform) which is a reference for calculating the degree of approximation, among the swept Doppler information (blood flow information or tissue exercise information), and superimposes and displays the reference Doppler information and the plurality of Doppler information items 310-1, 310-2, and 310-3 having a predetermined degree of approximation in the superimposing region 312, based on the degree of approximation calculated by the degree of approximation calculation unit 11. In addition, the Doppler information superimposing unit 10 may superimpose and display the plurality of Doppler information items 310-1, 310-2, and 310-3 which are most approximate to the reference Doppler information (Doppler waveform) among the swept Doppler information (blood flow information or tissue exercise information), in the superimposing region 312.

As a result, even when the heartbeat is not stable such as an irregular pulse, it is possible to extract Doppler information (Doppler waveform) corresponding to the stable heartbeat which is suitable for the measurement of the measurement values, and to observe the moving state of biological tissues (heart or a blood vessel) of the object based on the stable approximate Doppler information.

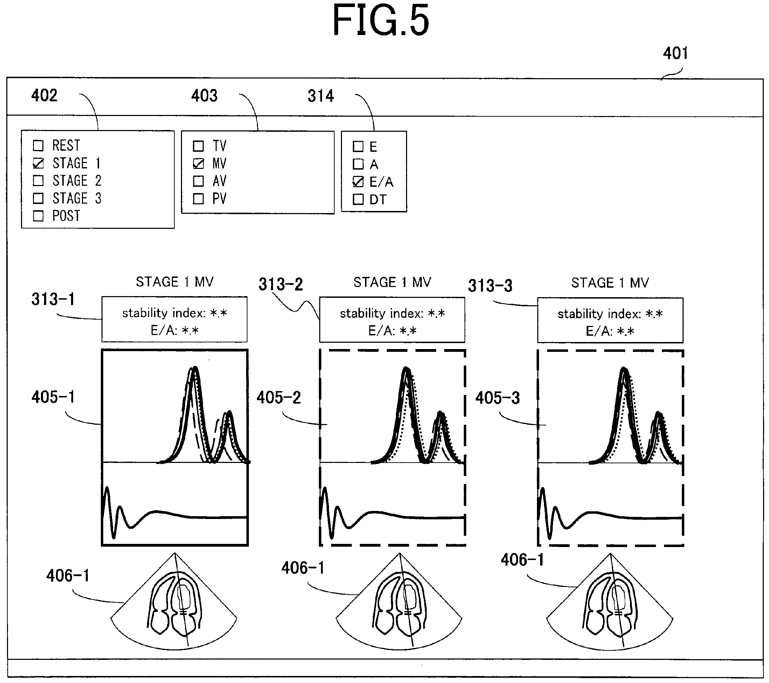

With the corresponding ultrasound image acquisition unit 8 and the approximate Doppler information acquisition unit 12, the approximate Doppler information (including the plurality of superimposed approximate Doppler information) of the plurality of sets and the corresponding ultrasound image are stored by the storage unit 17 and the approximate Doppler information representing a moving state of a biological tissue (heart or blood vessel) of the object 2 and showing useful characteristics for diagnosis may be selected from the plurality of sets. For example, the plurality of sets from the set having highest degree of approximation may be stored in the storage unit 17 and the set having highest degree of approximation may be selected therefrom. In addition, the plurality of sets may be displayed on the display unit 16 and the examiner may select the approximate Doppler information showing useful characteristics for diagnosis using a selection screen of the display unit 16. Fig. 5 is a diagram showing an example of a selection screen 401. As shown in Fig. 5, one set is selected from approximate Doppler information items 405-1, 405-2, and 405-3 of the plurality of sets. In Fig. 5, the stress stage 1 (STAGE1) is selected in a stage list 402 of the selection screen 401 and MV is selected in a part selection list 403. The approximate Doppler information items 405-1, 405-2, and 405-3 and the corresponding ultrasound images 406-1, 406-2, and 406-3 acquired in this condition are read out from the storage unit 17 and displayed on the selection screen 401. The approximate Doppler information items and the corresponding ultrasound images on the selection screen 401 are reproduced as a moving image, the Doppler waveform and the movement of the heart are synchronized to each other, and the observation can be performed. Stability index regions 313-1, 313-2, and 313-3 and a measurement value list 314 are displayed on the selection screen 401. The examiner can select the approximate Doppler information and the corresponding ultrasound image showing useful characteristics for diagnosis while observing the images, and can employ the approximate Doppler information and the corresponding ultrasound image as images used in the stress echocardiography. For example, a frame of the selected approximate Doppler information 405-1 may be displayed with a solid line and frames of the other approximate Doppler information 405-2 and 405-3 may be displayed with a broken line.

As a result, it is possible to select the approximate Doppler information representing a moving state of a biological tissue (heart or blood vessel) of the object 2 and showing useful characteristics for diagnosis, while observing the corresponding ultrasound image, stability index region (degree of approximation and determined results of the stability), and the measurement value, in addition to the approximate Doppler information.

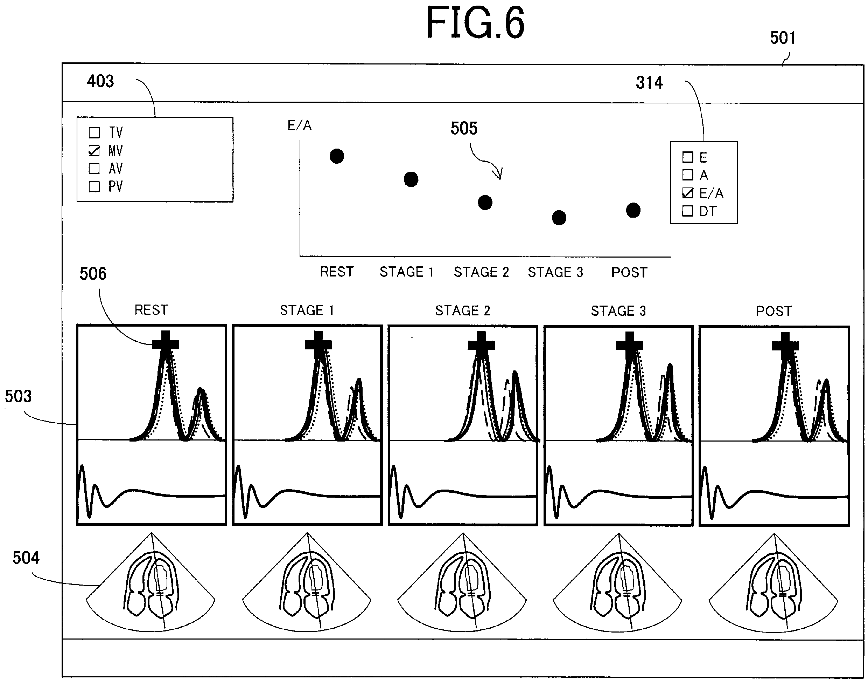

In addition, in order to observe the moving state (change in blood flow or the like) of the biological tissue (heart or blood vessel) of the object 2 before and after applying stress, the display unit 16 may display the approximate Doppler information (including the plurality of superimposed approximate Doppler information) and the corresponding ultrasound image corresponding to each stage of the stress echocardiography. Fig. 6 is a diagram showing an example of a display screen 501 on which the approximate Doppler information of each stage is displayed. As shown in Fig. 6, approximate Doppler information 503 of each stage, a corresponding ultrasound image 504, a measurement value region 505 of the measurement value, the part selection list 403, and the measurement value list 314 are displayed on the display screen 501. Herein, a cursor 506 showing the measurement part of the measurement value may be moved and the measurement value of the location of the cursor 506 may be reflected and displayed in the measurement value region 505 in real time.

As a result, it is possible to observe the moving state of a biological tissue (heart or blood vessel) of the object 2 while comparing the corresponding ultrasound image and the measurement value, in addition to the approximate Doppler information of each stage.

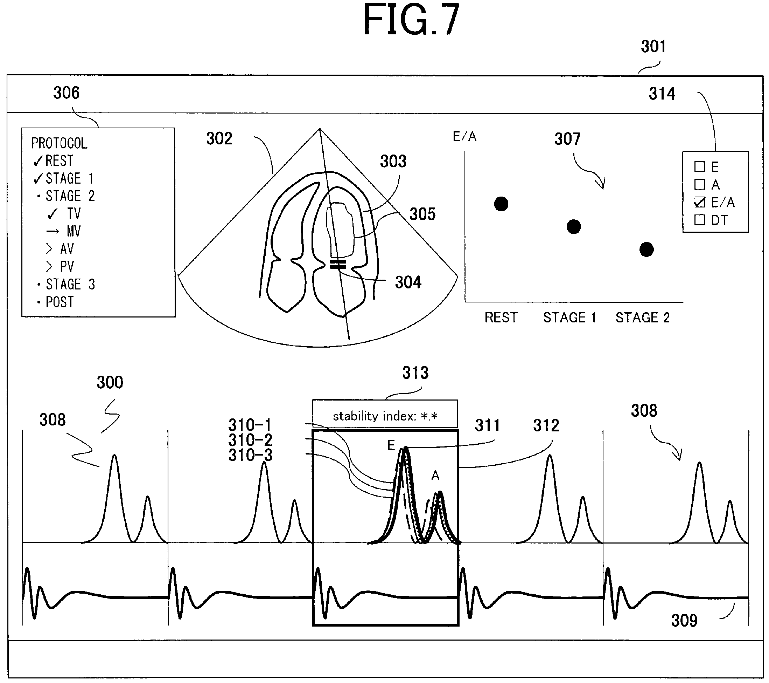

In addition, as shown in Fig. 7, the operation unit 18 may change the location of the superimposing region 312 set in the velocity distribution (blood flow information or tissue exercise information) 308 swept from the right to the left of the screen 301 over time. Fig. 7 is a diagram showing a change of the location of the heartbeat when acquiring the Doppler information. In Fig. 3, the Doppler information items corresponding to the plurality of heartbeats (three heartbeats) are superimposed in the order from the early input (first input) of the swept Doppler waveform (velocity distribution 308), and, when new Doppler information is input, the Doppler information superimposing unit 10 removes the oldest superimposed Doppler information, updates the plurality of Doppler information items, and superimposes and displays the information items in the superimposing region 312. In Fig. 7, the Doppler information at the arbitrary location (location of heartbeat) is superimposed, not in the order from the early input of the swept Doppler waveform (velocity distribution 308). The superimposing region 312 is moved by the operation unit 18. By moving the location of the superimposing region 312, the Doppler information superimposing unit 10 selects the reference Doppler information for calculating the degree of approximation from the temporarily continuous Doppler information items, and superimposes the plurality of Doppler information items including the reference Doppler information and displays the Doppler information items on the display unit. In Fig. 7, the superimposing region 312 is set in the center of the Doppler image (Doppler information) 300 of the velocity distribution. For example, the superimposing region 312 set in the center and the Doppler information corresponding to the three heartbeats before after the movement are superimposed. The movement operation of the superimposing region 312 may be performed in real time during the acquisition of the Doppler information or the ultrasound image and may be performed after acquiring the image or data. In addition, when the location of the superimposing region 312 is moved, the stability index region 313 or the measurement value region 307 in the moved location are updated and displayed as required.

As a result, it is possible to shorten the time until the swept Doppler waveform (velocity distribution 308) approaches the superimposing region 312. In addition, it is possible to acquire the Doppler information (velocity distribution 308) at an arbitrary location. Accordingly, it is possible to select the approximate Doppler information representing a moving state of a biological tissue (heart or blood vessel) of the object 2 and showing useful characteristics for diagnosis, while observing the swept Doppler information (velocity distribution 308).

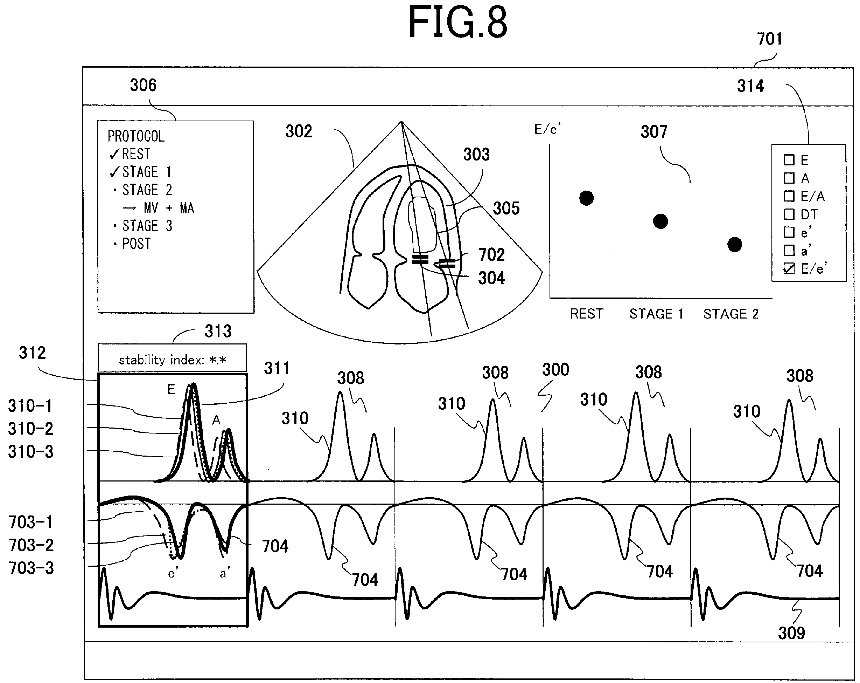

In addition, the Doppler information generation unit 5 may generate the Doppler information from the ultrasound signal of the plurality of parts of the object 2, the degree of approximation calculation unit 11 may calculate the degree of approximation of the Doppler information of the plurality of parts, and the approximate Doppler information acquisition unit 12 may acquire the approximate Doppler information of the Doppler information of the plurality of parts. The Doppler information generation unit 5 performs the Doppler operation of the plurality of parts set by the operation unit 18 and generates the Doppler information. Herein, a case of acquiring the two types of Doppler images (pulsed Doppler system and tissue Doppler system) will be described. Fig. 8 is a diagram showing the two types of Doppler operations performed by the Doppler information generation unit 5 and generation of the Doppler information. As shown in Fig. 8, the Doppler cursors 304 and 702 are set in MV and MA (mitral Annulus) which are the plurality of parts, and the Doppler information generation unit 5 acquires the mitral orifice blood flow velocity waveform and the mitral annulus exercise velocity waveform (Doppler information of the plurality of parts).

In the Doppler image (Doppler information) 300, the Doppler waveform (blood flow information) 310 of MV and the Doppler waveform (tissue exercise information) 703 of MA are displayed on the display unit 16 at the same time and are displayed in a line by being synchronized with the electrocardiogram 309. The measurement values of MV and MA are selected from the measurement value list 314. As a result, it is possible to evaluate the stability of the Doppler information and to observe the moving state of the biological tissues (heart or a blood vessel) of the object based on the stable approximate Doppler information.

Herein, the Doppler waveform (blood flow information) 310 of MV and the Doppler waveform (tissue exercise information) 703 of MA are displayed as an integrated Doppler information superimposed in the superimposing region 312, the degree of approximation of the plurality of Doppler information items is calculated as an integrated Doppler image, and the Doppler information items of plurality of parts are integrated and the stability of the Doppler information is evaluated. In this case, the Doppler information generation unit 5 generates the Doppler information from the ultrasound signals of the plurality of parts of the object 2, the degree of approximation calculation unit 11 calculates the degree of approximation by integrating the Doppler information items of the plurality of parts, and the approximate Doppler information acquisition unit 12 acquires the approximate Doppler information by integrating the Doppler information items of the plurality of parts.

In addition, regarding the Doppler waveform (blood flow information) 310 of MV and the Doppler waveform (tissue exercise information) 703 of MA, the degree of approximation of the Doppler information may be separately calculated and the stability of the Doppler information of the plurality of parts may be separately evaluated. As a result, it is possible to generally evaluate the stability of the Doppler information from the Doppler waveform (blood flow information or tissue exercise information) 310 of MV and the Doppler waveform (blood flow information or tissue exercise information) 703 of MA, and it is possible to generally observe the moving state of the biological tissues (heart or a blood vessel) of the object based on the stable approximate Doppler information. In this case, the Doppler information generation unit 5 generates the Doppler information from the ultrasound signal of the plurality of parts of the object 2, the degree of approximation calculation unit 11 calculates the degree of approximation in each of the plurality of parts, and the approximate Doppler information acquisition unit 12 acquires the approximate Doppler information in each of the plurality of parts.

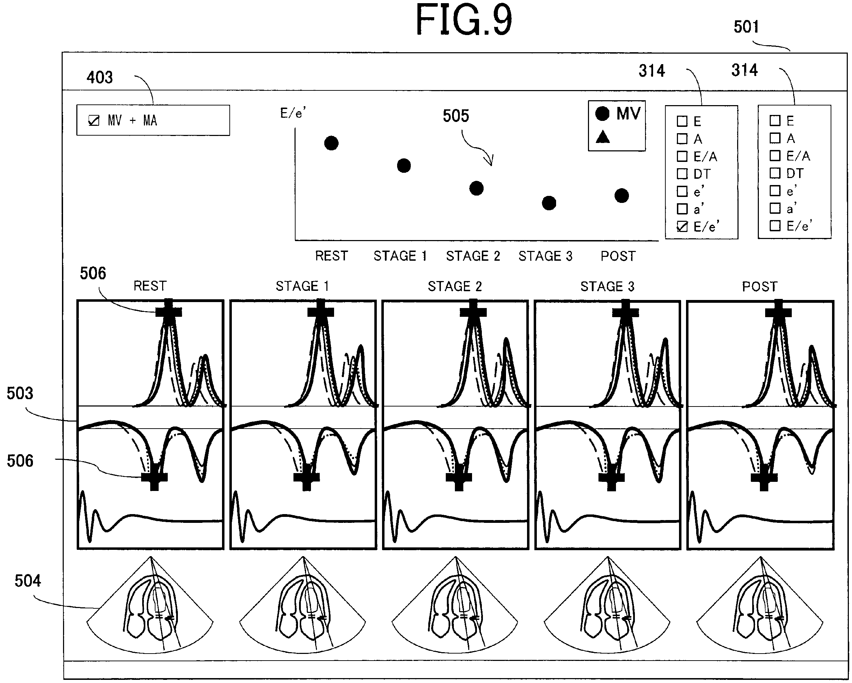

Fig. 9 is a diagram showing an example of the display screen 501 on which the approximate Doppler information of each stage is displayed, when the Doppler information is generated from the ultrasound signals of the plurality of parts. As shown in Fig. 9, regarding each of the plurality of parts, the approximate Doppler information 503 of each stage, the measurement value region 505 of the measurement value, the part selection list 403, and the measurement value list 314 are displayed on the display screen 501. In addition, the corresponding ultrasound image 504 of each stage is displayed on the display screen 501. Herein, regarding each of the plurality of parts, the cursor 506 showing the measurement part of the measurement value may be moved and the measurement value of the location of the cursor 506 may be reflected and displayed in the measurement value region 505 in real time. The cursor 506 can be set respectively for MV and MA.

As a result, regarding the each of the plurality of parts, it is possible to observe the moving state of a biological tissue (heart or blood vessel) of the object 2 while comparing the corresponding ultrasound image and the measurement value, in addition to the approximate Doppler information of each stage, it is possible to generally evaluate the stability of the Doppler information, and to generally observe the moving state of the biological tissues (heart or a blood vessel) of the object based on the stable approximate Doppler information.

### Industrial Applicability

The invention is advantageous for an ultrasound image pickup apparatus and an ultrasound image pickup method which can evaluate the stability of the Doppler information acquired by the Doppler ultrasonography and can observe the moving state of biological tissues (heart or a blood vessel) of an object based on the stabilized Doppler information.

### Reference Signs List

- 1: ULTRASOUND IMAGE PICKUP APPARATUS
- 3: ULTRASOUND SIGNAL GENERATION UNIT
- 4: ULTRASOUND IMAGE GENERATION UNIT
- 5: DOPPLER INFORMATION GENERATION UNIT
- 6: BIOLOGICAL SIGNAL GENERATION UNIT
- 7: DATA ACQUISITION UNIT
- 8: CORRESPONDING ULTRASOUND IMAGE ACQUISITION UNIT
- 9: DOPPLER MEASUREMENT UNIT
- 10: DOPPLER INFORMATION SUPERIMPOSING UNIT
- 11: DEGREE OF APPROXIMATION CALCULATION UNIT
- 12: APPROXIMATE DOPPLER INFORMATION ACQUISITION UNIT
- 13: PROTOCOL SETTING UNIT
- 14: PROTOCOL EXECUTION UNIT
- 15: OUTPUT UNIT
- 16: DISPLAY UNIT
- 17: STORAGE UNIT
- 18: OPERATION UNIT
- 19: CONTROL UNIT
- 60: STRESS DETERMINATION UNIT

## Claims

1. An ultrasound image pickup apparatus comprising:
a probe which transmits and receives an ultrasound signal to and from an object;
a Doppler information generation unit which generates Doppler information from the ultrasound signal;
a degree of approximation calculation unit which calculates a degree of approximation of the plurality of Doppler information items; and
an approximate Doppler information acquisition unit which acquires the Doppler information having a predetermined degree of approximation as approximate Doppler information.

2. The ultrasound image pickup apparatus according to claim 1, further comprising:
a Doppler information superimposing unit which superimposes the plurality of Doppler information items and displays the information items on a display unit.

3. The ultrasound image pickup apparatus according to claim 1,
wherein the degree of approximation calculation unit calculates at least one degree of approximation of a Doppler waveform of the plurality of Doppler information items synchronized with a biological signal, the time of the plurality of Doppler information items, and a measurement value based on the plurality of Doppler information items.

4. The ultrasound image pickup apparatus according to claim 1,
wherein the degree of approximation calculation unit enlarges or contracts a Doppler waveform of the Doppler information synchronized with a biological signal and calculates a degree of approximation of the Doppler waveform.

5. The ultrasound image pickup apparatus according to claim 1,
wherein the degree of approximation calculation unit calculates the degree of approximation based on at least one of blood flow information and tissue exercise information of the object.

6. The ultrasound image pickup apparatus according to claim 1,
wherein the degree of approximation calculation unit calculates a degree of approximation of the Doppler information based on at least one of difference, a ratio, a correlation coefficient, and pattern matching of the plurality of Doppler information items.

7. The ultrasound image pickup apparatus according to claim 1,
wherein the degree of approximation calculation unit calculates a degree of approximation of the plurality of Doppler information items according to the stress applied to the object.

8. The ultrasound image pickup apparatus according to claim 7, further comprising:
a stress determination unit which determines the stress based on a biological signal.

9. The ultrasound image pickup apparatus according to claim 1,
wherein the Doppler information superimposing unit superimposes the Doppler information as a reference and the plurality of Doppler information items having a predetermined degree of approximation and displays the information items on a display unit.

10. The ultrasound image pickup apparatus according to claim 1, further comprising:
a display unit which displays a plurality of sets of the approximate Doppler information in the descending order of the degree of approximation.

11. The ultrasound image pickup apparatus according to claim 1, further comprising:
a display unit which displays the approximate Doppler information corresponding to each stage of stress echocardiography.

12. The ultrasound image pickup apparatus according to claim 1,
wherein the Doppler information superimposing unit selects reference Doppler information for calculating the degree of approximation from the temporarily continuous Doppler information items, and
the ultrasound image pickup apparatus further includes a display unit which superimposes and displays the plurality of Doppler information items including the reference Doppler information.

13. The ultrasound image pickup apparatus according to claim 1,
wherein the Doppler information generation unit generates Doppler information from ultrasound signal of the plurality of parts of the object,
the degree of approximation calculation unit calculates the degree of approximation with the Doppler information items of the plurality of parts as an integrated item, and
the approximate Doppler information acquisition unit acquires the approximate Doppler information with the Doppler information items of the plurality of parts as an integrated item.

14. The ultrasound image pickup apparatus according to claim 1,
wherein the Doppler information generation unit generates Doppler information from ultrasound signal of the plurality of parts of the object,
the degree of approximation calculation unit calculates the degree of approximation in each of the plurality of parts, and
the approximate Doppler information acquisition unit acquires the approximate Doppler information in each of the plurality of parts.

15. An ultrasound image pickup method comprising:
transmitting and receiving an ultrasound signal to and from an object;
generating Doppler information from the ultrasound signal;
calculating a degree of approximation of the plurality of Doppler information items; and
acquiring the Doppler information having a predetermined degree of approximation as approximate Doppler information.
